# EUROPÄISCHE PATENTSCHRIFT

(11) **EP 0 576 868 B1**
(45) Veröffentlichungstag und Bekanntmachung des Hinweises auf die Patenterteilung: **05.04.2000**
(21) Anmeldenummer: 93109084.9
(22) Anmeldetag: 05.06.1993
(51) Int. Cl.: A61L 33/00, A61L 29/00

(54) **Medizinische Arbeitsmittel**
Medical articles
Articles médicaux

(30) Priorität: 02.07.1992 DE 4221665
(43) Veröffentlichungstag der Anmeldung: 05.01.1994
(73) Patentinhaber: REHAU AG + Co, 95111 Rehau (DE)
(72) Erfinder: Kühlein, Georg, W-8673 Rehau (DE)

(56) Entgegenhaltungen:
- EP-A- 0 136 916
- DE-A- 2 812 174
- GB-A- 1 013 235
- DERWENT JAPANESE PATENTS REPORT Week 6800, Derwent Publications Ltd., London, GB; AN 66-30851 & JP-A-43 003 291 (YOSHITOMI)

## Beschreibung

Die Erfindung betrifft die Verwendung eines Polymerweichmachers zur Herstellung medizinischer Arbeitsmittel wie Katheter, Schläuche, Behälter und Formteilen aus polymeren Werkstoffen, die eine besondere Erhöhung der Blutkompatibilität bei der Berührung mit Blut oder blutähnlichen Flüssigkeiten aufweisen, dadurch gekennzeichnet, dass
a) der Polymerweichmacher durch Acetylsalicylsäureester bzw.-mischester bzw.-teilester oder -amide verestert oder amidiert ist, oder
b) im Falle eines polymeren Polymerweichmachers der Kettenabschluss durch Acetylsalicylsäureester bzw. -mischester bzw. - teilester oder -amide gebildet ist.

Aus der DE-A 19 30 136 ist ein beschichteter Katheter bekannt, welcher als Basis aus einem Schlauch aus Gummi oder einem Elastomeren besteht. Dieser Katheterschlauch ist an der Außen- und/oder Innenwand mit einem hydrophilen Polyacrylat bzw. -Methacrylat, insbesondere mit einem Hydroxialkyl-oder einem Hydroxialkoxi-Alkyl-Acrylat bzw. -Methacrylat mit jeweils niederen Akylresten beschichtet.

Weiterhin ist es bekannt, derartige medizinische Arbeitsmittel mit einer Beschichtung aus Heparin zu versehen. Diese Heparin-Beschichtungsverfahren arbeiten über APTES- und TDMAC-Brücken. Mit dieser Heparin-Beschichtung wird das Anhaften von Blutplättchen an der Oberfläche des medizinischen Arbeitsmittels verhindert und gleichzeitig die intrinsische Koagulation desaktiviert. Ausführungen zum Beschichtungsverfahren mit Heparin über TDMAC-Brücken finden sich bei G.A. Grode, R.D. Falb, J.P. Crowley in J. Biomed. Mater. Res. Symp., 3, 77 (1972). Entsprechende Hinweise zur Beschichtung mit Heparin über APTES-Brücken finden sich bei R.L. Merker, L.J. Elyasch, S.W. Mayhew, J.Y.C. Wang in Proc. Artif. Heart Conf., 1969, Seite 29.

Alle diese Verfahren haben den prinzipiellen Nachteil, daß sie technisch aufwendig und sehr arbeitsintensiv sind. So läuft eine Heparinisierung bei beiden genannten Verfahren in mehreren zusätzlichen Arbeits- und Beschichtungszyklen ab.

Ein weiterer wesentlicher Nachteil dieser bekannten Verfahren ist, daß die auf diese Weise hergestellten medizinischen Halbzeuge sterilisiert werden müssen, weil die Beschichtung mit Heparin mikrobiell abgebaut werden kann. Da nach der Konfektion der Halbzeuge zu Fertigprodukten eine Gesamtsterilisation erforderlich ist, ist hier als weiterer Nachteil das erhebliche Risiko einer Mehrfachsterilisation zu nennen.

Hier setzt die Erfindung ein, die es sich zur Aufgabe gestellt hat, die zum Stand der Technik genannten Nachteile zu vermeiden und eine Methode zur Erzielung der optimalen Blutkompatibilität bei medizinischen Halbzeugen und Fertigprodukten aufzuzeigen, bei der über das medizinische Arbeitsmittel weder das biologische System negativen Auswirkungen ausgesetzt ist noch das Material, aus dem das medizinische Arbeitsmittel gefertigt ist durch Einwirkung des biologischen Systems so weit geschädigt werden kann, daß es die vorgesehene Funktion nicht mehr erfüllt. Erfindungsgemäß wird dazu vorgeschlagen, daß in das Ausgangspolymere vor der eigentlichen Formgebung eine definierte Menge von Acetylsalicylsäureestern bzw -mischestern als Zuschlagstoffe eingemischt sind.

Die Erfindung geht von der Überlegung aus, dem Ausgangspolymeren alsAdditive Acetylsalicylsäureester bzw. -mischester zuzusetzen, die den Werkstoff von der Blutkompatibilität her mit seinem relativen Gerinnungsparameter dem Quotienten 1,0 annähern. So hat es sich bei der Verwendung von weichmacherhaltigem polyvinylchlorid als Ausgangsmaterial für die Herstellung medizinischer Arbeitsmittel als vorteilhaft herausgestellt, daß die heute für solche Anwendungszwecke üblichen PVC-Rezepturen auf Basis Zink und Kalzium erfindungsgemäß durch die genannten Acetylsalicylsäureester bzw. - mischester ergänzt werden. Hierbei hat sich als zweckmäßig eine Dosierungsrate von 0,01 bis 2,0 Gewichtsprozent erwiesen.

Bei Verwendung von reiner Acetylsalicylsäure der Formel 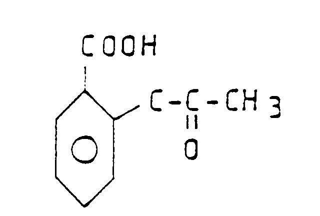 hat sich herausgestellt, daß eine Dosierungsrate von 2 Gewichtsprozent 100 Gewichtsprozent des Ausgangspolymeren als optimale Dosierungsrate zugemischt werden.

In bestimmten Fällen kann es zweckmäßig sein, anstatt der reinen Acetylsalicylsäure, Acetylsalicylsäureester oder Acetylsalicylsäure-Mischester als Zuschlagsstoff einzusetzen. Derartige Ester bzw. Mischester nach der Formel 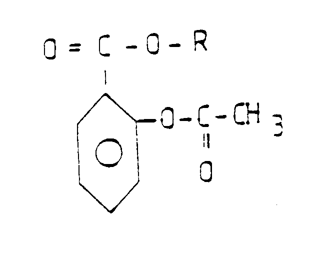 können in einem Mengenanteil zwischen 0,01 und 25 Gewichtsprozent dem Ausgangspolymeren beigemischt werden, wobei die Zumischung der Gewichtsprozentanteile des Acetylsalicylsäure-Mischesters jeweils zu 100 Gewichtsprozent des Ausgangspolymeren erfolgt.

Es hat sich als zweckmäßig erwiesen, daß die Acetylsalicylsäure mit gesättigten und/oder ungesättigten Fettalkoholen C₂ bis C₂₄ gemäß der Formel 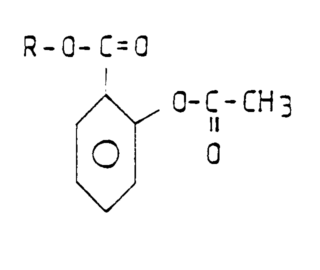 verestert ist. Bei dieser Formel ist R das Synonym für die gesättigten und/oder ungesättigten Fettalkohole C₂ bis C₂₄.

Gemäß einem Heiteren Ausführungsbeispiel kann die Acetylsalicylsäure mit einem Glyzerin gemäß der Formel 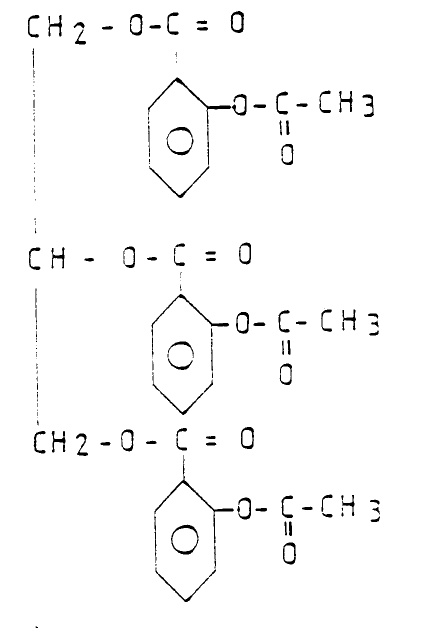 verestert sein.

Eine weitere Möglichkeit besteht erfindungsgemäß darin, die Acetylsalicylsäure mit Polyethylenglykol gemäß der Formel 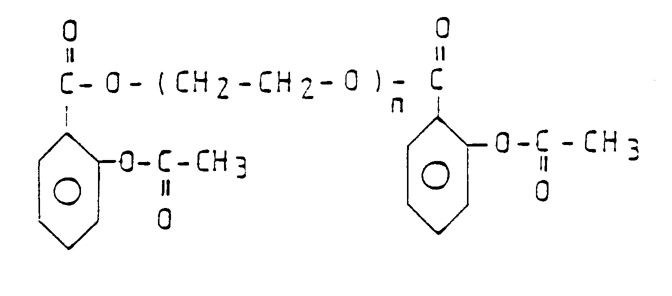 zu verestern, wobei n = 3 bis 25000 ist.

Weitere Veresterunsmöglichkeiten bestehen darin, daß die Acetylsalicylsäure mit Pentaerythit gemäß der nachfolgenden Formel 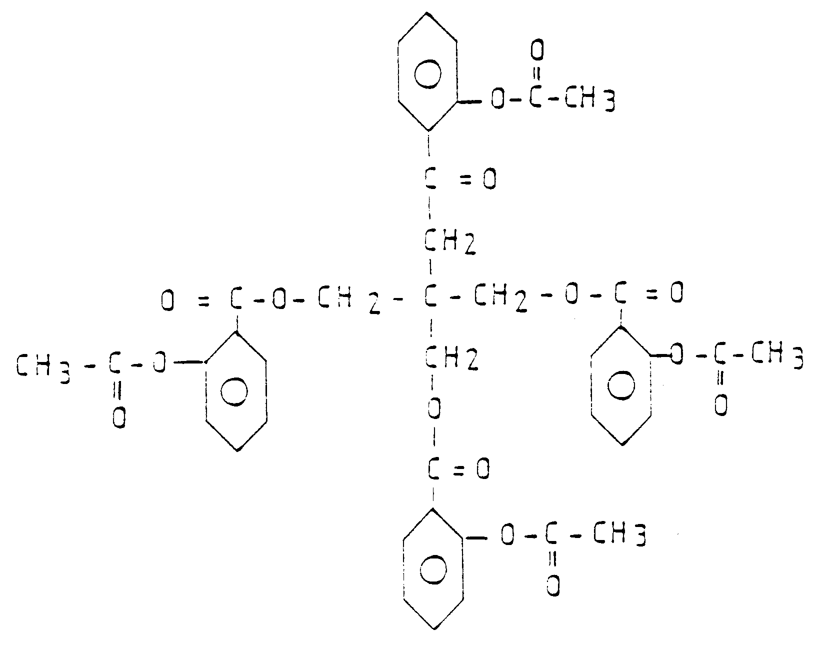 verestert ist.

Des weiteren kann die Acetylsalicylsäure erfindungsgemäß mit Glykol gemäß der Formel 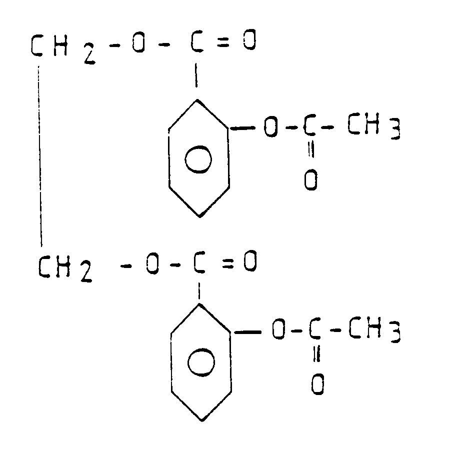 verestert sein und schließlich kann die Veresterung der Acetylsalicylsäure mit Neopentylalkohol gemäß der Formel 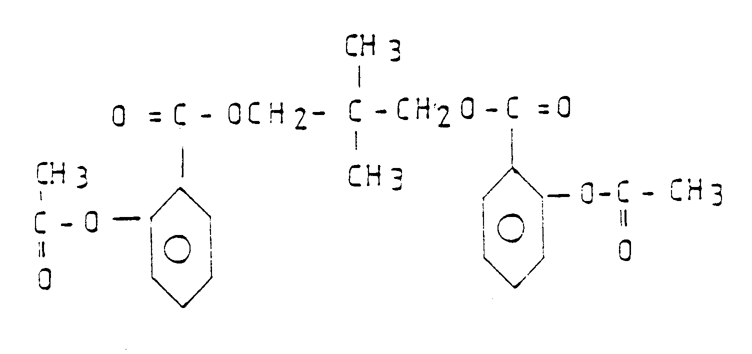 erfolgen.

Weiter kann die Acetylsalicylsäure über Amidgruppen an aminogruppenhaltige Substanzen entsprechend der nachfolgenden Formel 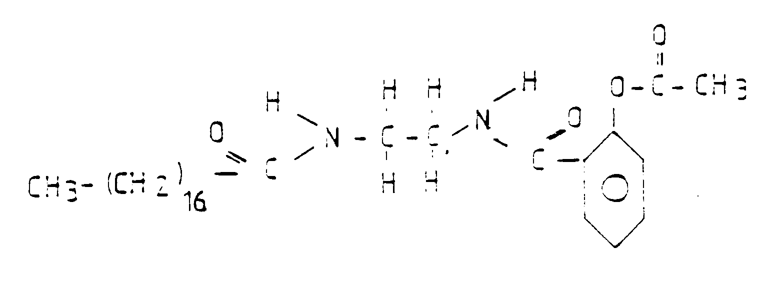 gebunden werden. Damit werden die Gleiteigenschaften von mit dieser Verbindung versetzten Polymerschmelzen verbessert.

Die vorgenannten Beispiele zeigen die erfindungsgemäße Möglichkeit, Ausgangspolymere vor der eigentlichen Formgebung mit Acetylsalicylsäureestern bzw. -mischestern als Zuschlagstoffe auszustatten. Diese Zuschlagstoffe können andere Zuschlagsmaterialien entsprechend ergänzen. Das wesentliche Merkmal dieser erfindungsgemäß zu verwendenden Zuschlagstoffe wird jedoch darin gesehen, daß die damit ausgestatteten medizinischen Arbeitsmittel die Blutplättchen-Aggregation verhindern und die intrinsische Koagulation desaktivieren. Eine Vorsterilisierung der Halbzeuge ist nicht mehr erforderlich, da deren erfindungsgemäße Ausstattung entgegen der bisher üblichen Heparin-Beschichtung nicht mehr mikrobiell abgebaut werden kann.

Neben der direkten Zumischung der Acetylsalicylsäureester bzw. -mischester zu dem Ausgangspolymeren können diese Acetysalicylsäureester bzw. -mischester bzw. -teilester den Kettenabschluß eines Polymerweichmachers darstellen. Auf diese weise lassen sich die üblichen Weichmacher bereits erfindunasgemäß ausstatten. die dann in der rezepturüblichen Menge dem Ausgangspolymeren beigemischt werden.

In der nachstehenden Formel ist als Beispiel ein Polymerweichmacher basierend auf Adipinsäure und 1,3 Butandiol endstandig mit Acetylsalicylsäure terminiert:

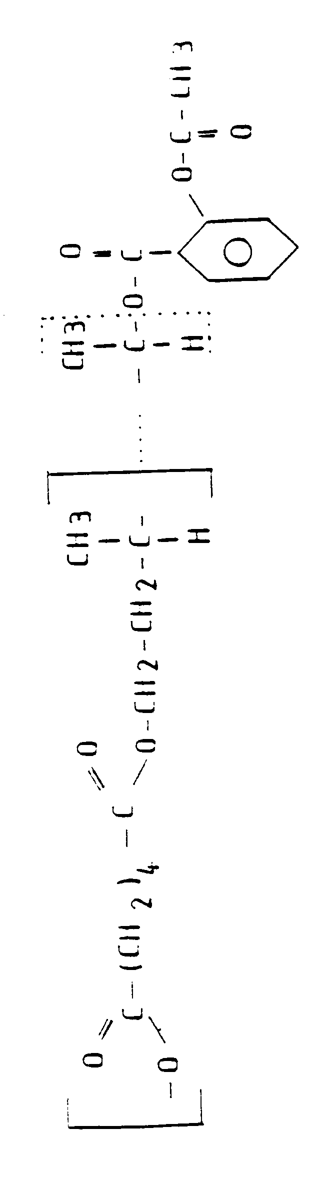

Die nachfolgenden Beispiele zeigen die Verwendungsmöglichkeit der erfindungsgemäß zu verwendenden Acetylsalicylsäureester bzw. -mischester bzw. -teilester als Zuschlagstoffe für polymere Materialien zur Herstellung medizinischer Arbeitsmittel.

### Beispiel 1:

### Weich-PVC-Rezeptur

| | | |
|---|---|---|
| 1.1 | 100 | Teile S-PVC |
| | 10-90 | Teile Weichmacher |
| | 1-10 | Teile epoxidierte Öle |
| | 0,1-1 | Teile Metallseifen |
| | 0,1-1,5 | Teile Gleitmittel |
| | 0,2-2 | Teile Glycerin tri (Acetylsalicylat) |
| 1.2 | 100 | Teile S-PVC |
| | 10-90 | Teile Polyglycoladipat, Acetylsalicylsäure-terminiert |
| | 1-10 | Teile epoxidierte Öle |
| | 0-90 | Teile Weichmacher |
| | 0,1-1 | Teile Metallseifen |
| | 0,1-1,5 | Teile Gleitmittel |

### Beispiel 2:

### Polyurethan-Rezeptur

| | |
|---|---|
| 100 Teile | Polyurethan |
| 0,5-20 Teile | Polyglycoladipat, Acetylsalicylsäure-terminiert |

### Beispiel 3:

### Polyethylen-Rezeptur

| | |
|---|---|
| 100 Teile | Polyethylen |
| 0,01-5 Teile | N-Stearoyl-N'Acetylsalicylsäureethylendiamin |

### Beispiel 4:

### Polypropylen-Rezeptur

| | |
|---|---|
| 100 Teile | Polypropylen |
| 0,01-5 Teile | Ester von Acetylsalicylsäure und C 12 bis C 24 Fettalkoholen |

### Beispiel 5:

### Weichmacher-Rezeptur

| | |
|---|---|
| 10 - 90 Teile | Polymerweichmacher, Acetylsalicylsäure-terminiert |
| 0 - 90 Teile | Weichmacher |

## Patentansprüche

1. Verwendung eines Polymerweichmachers zur Herstellung medizinischer Arbeitsmittel wie Katheter, Schläuche, Behälter und Formteile aus polymeren Werkstoffen, die eine besondere Erhöhung der Blutkompatibilität bei der Berührung mit Blut oder blutähnlichen Flüssigkeiten aufweisen, dadurch gekennzeichnet, dass
a) der Polymerweichmacher durch Acetylsalicylsäureester bzw. -mischester bzw. -teilester oder -amide verestert oder amidiert ist
oder
b) im Falle eines Polymeren Polymerweichmachers der Kettenabschluss durch Acetylsalicylsäureester bzw. -mischester bzw. -teilester oder -amide gebildet ist.

2. Verwendung nach Anspruch 1, dadurch gekennzeichnet, daß die Acetylsalicylsäure mit gesättigten und/oder ungesättigten Fettalkoholen C₂ bis C₂₄ verestert ist.

3. Verwendung nach Anspruch 1, dadurch gekennzeichnet, daß die Acetylsalicylsäure mit Glyzerin verestert ist.

4. Verwendung nach Anspruch 1, dadurch gekennzeichnet, daß die Acetylsalicylsäure mit Polyethylenglykol verestert ist.

5. Verwendung nach Anspruch 1, dadurch gekennzeichnet, daß die Acetylsalicylsäure mit Pentaerythrit verestert ist.

6. Verwendung nach Anspruch 1, dadurch gekennzeichnet, daß die Acetylsalicylsäure mit Glykol verestert ist.

7. Verwendung nach Anspruch 1, dadurch gekennzeichnet, daß die Acetylsalicylsäure mit Neopentrylalkohol verestert ist.

8. Verwendung nach Anspruch 1, dadurch gekennzeichnet, daß die Acetylsalicylsäure über eine Amidgruppe an eine aminogruppenhaltige Substanz gebunden ist.

9. Verwendung nach Anspruch 1, dadurch gekennzeichnet, daß der Acetylsalicylsäureanteil zwischen 0,01 und 25 Gewichtsprozent liegt.

## Claims

1. Use of a polymer plasticizer to produce medical supplies, such as catheters, tubing, vessels and molded parts of polymer materials, which have particularly high blood compatibility upon contact with blood or blood-like fluids, is characterized in that a) the polymer plasticizer is esterified or amidated by means of esters, mixed esters, partial esters or amides of acetylsalicylic acid or b) in case of a polymeric polymer plasticizer, the chain end is formed by means of esters, mixed esters, partial esters or amides of acetylsalicylic acid.

2. Use according to Claim 1 is characterized in that the acetylsalicylic acid is C₂ to C₂₄ esterified using saturated and/or unsaturated fatty alcohols.

3. Use according to Claim 1 is characterized in that the acetylsalicylic acid is esterified using glycerin.

4. Use according to Claim 1 is characterized in that the acetylsalicylic acid is esterified using polyethylene glycol.

5. Use according to Claim 1 is characterized in that the acetylsalicylic acid is esterified using pentaerythritol.

6. Use according to Claim 1 is characterized in that the acetylsalicylic acid is esterified using glycol

7. Use according to Claim 1 is characterized in that the acetylsalicylic acid is esterified using neopentylalcohol,

8. Use according to Claim 1 is characterized in that the acetylsalicylic acid is bonded to a substance containing an amino group using an amide group.

9. Use according to Claim 1 is characterized in that the acetylsalicylic acid portion lies between 0.01 and 25 weight percent.

## Revendications

1. Utilisation d'un plastifiant polymère pour la fabrication de matériel médical tel que cathéters, tubulures, réservoirs et pièces moulées en polymères, présentant une augmentation de la compatibilité sanguine lors de contacts avec du sang ou des liquides apparentés au sang, caractérisée en ce que
a) le plastifiant polymère est transestérifié par des esters/esters mélangés/esters partiels d'acide acétylsalicilique ou amidifié par des amides d'acide acétylsalicilique ou
b) dans le cas d'un plastifiant polymère, la terminaison de la chaîne est formée par un ester/ester mélangé/ester partiel d'acide acétylsalicilique ou un amide d'acide acétylsalicilique.

2. Utilisation selon la revendication 1, caractérisée en ce que l'acide acétylsalicilique est estérifié avec des alcools gras C₂ à C₂₄ saturés et/ou non saturés.

3. Utilisation selon la revendication 1, caractérisée en ce que l'acide acétylsalicilique est estérifié avec de la glycérine.

4. Utilisation selon la revendication 1, caractérisée en ce que l'acide acétylsalicilique est estérifié avec du polyéthylène-glycol.

5. Utilisation selon la revendication 1, caractérisée en ce que l'acide acétylsalicilique est estérifié avec du pentaérythritol.

6. Utilisation selon la revendication 1, caractérisée en ce que l'acide acétylsalicilique est estérifié avec du glycol.

7. Utilisation selon la revendication 1, caractérisée en ce que l'acide acétylsalicilique est estérifié avec de l'alcool néopentylique.

8. Utilisation selon la revendication 1, caractérisée en ce que l'acide acétylsalicilique est combiné à une substance contenant un groupement aminé par le biais d'un groupe d'amides.

9. Utilisation selon la revendication 1, caractérisée en ce que la part d'acide acétylsalicilique représente de 0,01 à 25 % du poids.
